# FASCICULE DE BREVET EUROPEEN

(11) **EP 1 267 768 B1**
(45) Date de publication et mention de la délivrance du brevet: **28.12.2005**
(21) Numéro de dépôt: 01909911.8
(22) Date de dépôt: 27.02.2001
(51) Int. Cl.: A61F 13/08

(54) **ORTHESE DE CONTENTION DE TYPE PLACEBO**
KOMPRESSIONSSTRUMPF ALS PLACEBO
PLACEBO-TYPE COMPRESSION ORTHESIS

(30) Priorité: 29.02.2000 FR 0002527
(43) Date de publication de la demande: 02.01.2003
(73) Titulaire: Innothera Topic International (Societe Anonyme), 94110 Arcueil (FR)
(72) Inventeur: GROSJEAN, Eric, F-88000 Epinal (FR); MATHIEU, Joel, F-88510 Eloyes (FR); GARDON-MOLLARD, Christian, F-63400 Chamalières (FR)
(74) Mandataire: Dupuis-Latour, Dominique
(86) Numéro de dépôt international: PCT/FR2001/000567
(87) Numéro de publication internationale: WO 2001/064152

(56) Documents cités:
- FR-A- 688 378
- FR-A- 1 122 832
- FR-A- 2 017 339
- FR-A- 2 432 867
- FR-A- 2 588 890
- FR-A- 2 654 925
- US-A- 4 172 456
- US-A- 4 422 307
- US-A- 4 520 635
- US-A- 4 522 044
- US-A- 4 527 402
- US-A- 4 745 917

## Description

L'invention a trait au domaine des orthèses compressives d'un ou des deux membres inférieurs, généralement connues sous la dénomination "bas de contention" ou "collants de contention".

Bien que dans la suite on utilisera le terme "bas", l'invention n'est pas limitée à un article particulier, mais s'applique aussi bien à toutes les orthèses compressives, qu'il s'agisse de collants, de mono-collants, de bas ou de chaussettes.

On oppose classiquement les "bas de contention", qui sont des bas médicaux produisant un effet thérapeutique par compression/contention des membres inférieurs, aux "bas de maintien" (ou encore "bas de soutien" ou "bas anti-fatigue") et aux "bas mode". Les bas de contention médicaux sont des bas qui exercent une pression, mesurée à la cheville, de 10 à plus de 36 mmHg (13 à 48 hPa ; on utilisera toutefois dans la présente description le mmHg comme unité de mesure de pression compte tenu de son usage universel dans le domaine de la phlébologie et de la contention médicale). De plus, les bas de contention exercent une pression dégressive de la cheville vers la cuisse.

Les FR-A-2 654 925 et FR-A-2 588 890 décrivent de telles orthèses susceptibles d'appliquer une pression de contention effective à effet thérapeutique.

Les bas de maintien n'exercent à la cheville qu'une pression de 6 à 14 mmHg et les bas mode, de 1 à 4 mmHg.

Les bas de contention, pour permettre une compression forte des membres inférieurs, sont réalisés en un matériau élastique, typiquement une maille tricotée de texture très serrée avec, en outre, incorporation d'un fil de trame élastique (généralement un élasthanne guipé) de titre élevé.

Cette structure particulière donne au bas de contention un aspect visuel et tactile particulier et reconnaissable, très différent de celui des bas de maintien, et *a fortiori* des bas mode.

L'un des buts de l'invention est de proposer une structure de produit tricoté présentant sensiblement la même morphologie, le même aspect et le même toucher qu'un bas médical de contention mais qui, au contraire de ce dernier, n'exerce sur les membres inférieurs qu'une pression insuffisante pour provoquer un effet thérapeutique notable.

L'utilité d'un tel produit est celle d'un "placebo textile" pour des essais cliniques destinés à démontrer les effets thérapeutiques des bas médicaux de contention, en suivant une méthodologie du type "essai *versus* placebo en double insu" telle que celle employée couramment pour mesurer les effets de principes actifs médicamenteux.

Une telle étude *versus* placebo est par exemple décrite par M. Chauveau et F. Agbomson, "Force de compression et symptomatologie de l'insuffisance veineuse fonctionnelle des membres inférieurs : efficacité comparée de six degrés de contention", *Phlébologie* 1997;50:731-736.

Cette étude avait toutefois été réalisée pour démontrer l'efficacité de bas de maintien, auxquels il est aisé de substituer un bas placebo sous la forme d'un bas mode de même couleur et de même texture. En effet, bas mode et bas de maintien sont visuellement et tactilement très proches entre eux et peuvent être substitués aisément en respectant les impératifs d'un essai croisé en double insu.

Tel n'est pas le cas des véritables bas de contention, qui sont visuellement et tactilement très différents des bas de maintien.

Un essai *versus* placebo en double insu impose donc de disposer d'un produit ayant le même aspect visuel et le même toucher qu'un bas actif mais avec une compression négligeable, qui ne puisse pas être *a priori* distingué du bas actif ni par le patient ni par le médecin prescripteur de la contention placebo afin de respecter les procédures du double insu.

Pour présenter une similitude visuelle et tactique avec un bas de contention, un bas placebo doit être tricoté avec la même structure qu'un bas de contention (maille jersey ou micromesh combinée à un fil de trame élastique). Mais au contraire de ce dernier, il doit procurer une pression à la cheville inférieure à 5 mmHg environ (on montre en effet que les phénomènes hémodynamiques commencent à environ 7 mmHg).

En outre et de préférence, à la différence du bas de contention, le bas placebo ne devra pas être dégressif, c'est-à-dire que la pression à la cuisse devra être du même ordre que la pression à la cheville, typiquement un ratio de dégressivité cuisse/cheville compris entre 0,8 et 1,1. Il y a lieu toutefois de noter que, pour des faibles valeurs de pression, les écarts entre cheville et cuisse sont souvent inférieurs à la précision des instruments de contrôle, de sorte qu'il est en pratique difficile de vérifier avec précision les résultats obtenus concernant ce paramètre.

Au surplus, pour des raisons évidentes de commodité et de coût de fabrication, il est souhaitable que les bas placebo puissent être tricotes sur les mêmes machines que les bas de contention, sans modification substantielle de la programmation de ces dernières.

Ces impératifs multiples et souvent contradictoires sont résolus, selon l'invention, en réalisant un bas par tricotage d'un fil de tricot combiné à un fil de trame extensible, ce bas étant un bas de type placebo apte à exercer au niveau de la cheville une pression de contention inférieure à 8 mmHg, de préférence inférieure à 5 mmHg, et dans lequel:
- le fil de trame présente une masse linéique, mesurée sans tension, comprise entre 250 et 600 dtex, de préférence comprise entre 350 et 500 dtex ; le fil de trame présente une proportion de matériau élastique inférieure à 25 %, de préférence inférieure à 20 %, du titre total du fil ; et le fil de trame présente une capacité d'élongation comprise entre 200 et 350 %, de préférence comprise entre 200 et 300 %.

Selon diverses autres caractéristiques de l'invention :
- le rapport des pressions de contention entre cuisse et cheville est inférieur à 125 %, de préférence inférieur à 110 % ; et
- le rapport des pressions de contention entre cuisse et cheville est au moins égal à 80 % ;

On va maintenant décrire plusieurs exemples de mise en oeuvre de l'invention, en référence aux dessins annexés.
La figure 1 montre la structure générale de la maille d'un bas de contention.
La figure 2 montre les caractéristiques d'allongement comparatives d'un bas de contention véritable et d'un bas placebo selon l'invention.

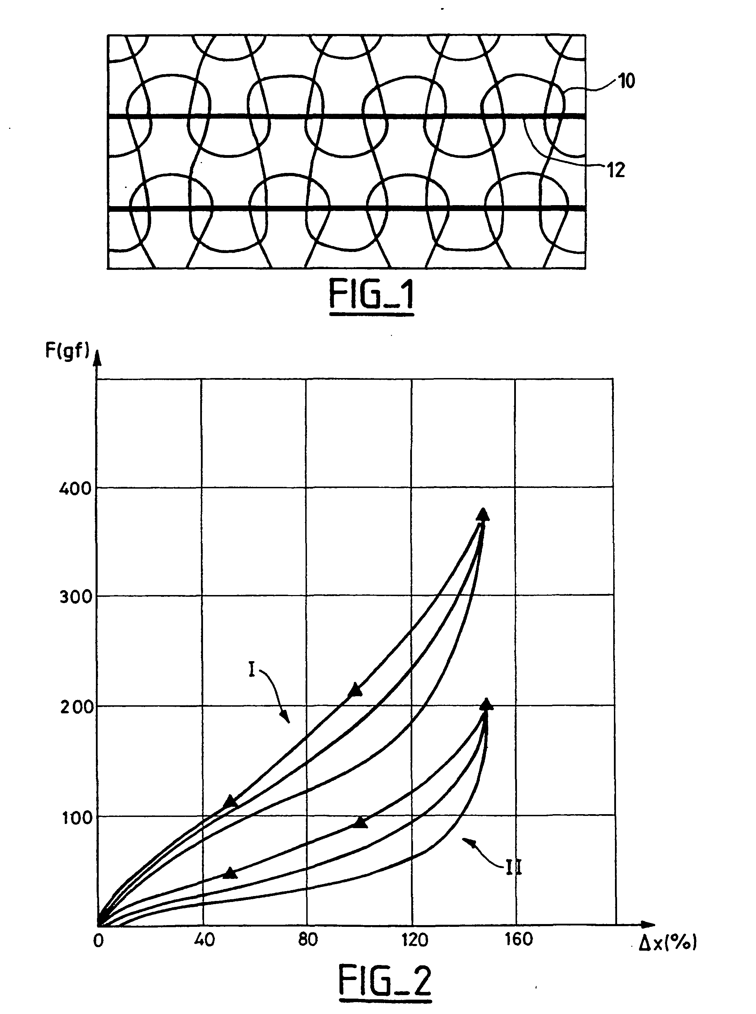

Le bas placebo selon l'invention est réalisé avec une structure illustrée figure 1, semblable à celle d'un bas de contention classique.

Il s'agit d'une structure tricotée utilisant un fil de tricot 10, par exemple en maille jersey, combiné à un fil de trame 12 en élasthanne guipé procurant la contention recherchée.

L'invention porte sur le choix particulier du fil de trame 12, qui doit permettre à l'article d'être visuellement et tactilement semblable à un bas de contention classique, tout en présentant des propriétés de compression et de dégressivité radicalement différentes de ce dernier.

On va décrire ci-dessous une série d'exemples mettant en oeuvre divers choix de fils conduisant à des propriétés très différentes.

### Exemple 1

À titre de référence, on a réalisé un bas de contention classique de classe I (classe française) avec les fils suivants.

Le fil de tricot (qui sera le même dans tous les exemples ci-après) est un fil d'élasthanne Lycra (marque déposée de Dupont de Nemours) de 17 dtex guipé PA 6.6 FTS 42/46 brins (guipage par un fil polyamide 42 dtex avec fausse torsion dans le sens S).

Le fil de trame est un fil d'élasthanne guipé double recouvrement présentant les caractéristiques suivantes :
- âme élasthanne 310 dtex
- étirage 1/3,3
- couverture inférieure (CI) PA 6.6 FTZ 1/22/7 (polyamide 6.6 fausse torsion sens Z 1 fil 22 dtex 7 brins)
- couverture supérieure (CS) PA 6.6 FTZ 1/22/7.

Le fil obtenu présente un titre de 400 dtex, avec 62 % d'élasthanne pour 38 % de polyamide. Sa capacité d'élongation est de l'ordre de 250 à 300 %.

Le fil ainsi obtenu présente une caractéristique d'allongement illustrée en 1 sur la figure 2, qui donne la force en fonction du pourcentage de déformation (la caractéristique présente une certaine hystérésis naturelle). Cette caractéristique a été mesurée au moyen d'un dynamomètre à gradient d'allongement constant sur une spire de fil de 2 m de circonférence doublée huit fois (la force exercée sur une section du fil est donc 1/16^{e} de la force exercée par le dynamomètre), le fil étant étiré jusqu'à 150 %.

Le résultat obtenu est une indication visuelle de la "nervosité" (raideur) du fil, d'autant plus élevée que le pourcentage d'élasthanne dans le titre du fil est élevé.

À partir des deux fils décrits ci-dessus, on a tricoté un bas présentant les caractéristiques suivantes :
- largeur : 10,7/15/19,5 mm (largeurs respectivement à la cheville, au mollet et à la cuisse)
- longueur : 125/25/68 mm (idem).

Un tel bas, après formage et teinture selon les procédés classiques, procure une pression à la cheville supérieure à 10 mmHg, avec une dégressivité cheville/cuisse de 75 %, conforme donc aux prescriptions d'un bas de contention de classe I.

### Exemple 2

On a tout d'abord réalisé, pour mémoire, un bas dépourvu de fil de trame 12. Un tel bas, certes, ne délivre pratiquement aucune pression de contention (de l'ordre de 1 mmHg). Mais du fait de l'absence de fil de trame élastique, cette texture, immédiatement reconnaissable par son manque de tenue, se rapproche de celle d'un bas mode et n'est donc pas appropriée à l'usage recherché.

### Exemple 3

Dans cet exemple, on a cherché à réduire le titre d'élasthanne proportionnellement à la baisse recherchée pour la pression de contention.

Ainsi, par rapport aux bas de contention de classe I de l'exemple 1 délivrant une pression à la cheville de 10 mmHg, on a cherché à diviser par deux le titre du fil d'élasthanne, en compensant la différence par une augmentation de la proportion de polyamide.

Une condition essentielle à l'obtention d'un bas placebo satisfaisant est en effet qu'il soit réalisé à partir d'un fil présentant une masse linéique proche de celle d'un fil entrant dans la composition d'un bas de contention classique, pour conserver le même aspect visuel.

Une autre condition est que le fil du bas placebo offre une capacité d'élongation identique à celle d'un fil de bas de contention, afin qu'il puisse être tricoté dans les mêmes conditions que ce dernier.

On a ainsi substitué, toutes choses égales par ailleurs, au fil de trame de l'exemple 1 (âme élasthanne 310 dtex pour un titre au repos de 400 dtex) un fil présentant la composition suivante :
- âme élasthanne 156 dtex
- étirage 1/2,65
- CI PA 6.6 FT1/33/20
- CS PA 6.6 FT1/33/20.

Le bas est tricoté de la même manière que précédemment.

Après formage et teinture, on mesure une pression à la cheville de 5,2 mmHg et une pression de cuisse de 3,72 mmHg de mercure, soit une dégressivité de 71 %.

Le bas ainsi réalisé est satisfaisant sur le plan de la pression à la cheville, mais non sur celui de la dégressivité, trop éloignée de la valeur idéale de 100 % (en pratique, on considère que la valeur doit être comprise entre 0,8 et 1,1 pour être satisfaisante).

Des essais ont été menés en jouant sur l'étirage du fil d'élasthanne 156 dtex, mais n'ont pas permis d'aboutir à un résultat satisfaisant. En diminuant la valeur d'étirage à 1/2,5, la capacité d'élongation du fil n'est en effet pas suffisante pour réaliser en pratique le collant.

### Exemple 4

Pour pallier les limitations rencontrées plus haut, on a abaissé encore le titre du fil d'élasthanne.

Dans cet exemple, on a choisi pour le fil de trame la composition suivante :
- âme élasthanne 78 dtex
- étirage 1/3,5
- CI PA 6.6 FT 2/33/20
- CS PA 6.6 FT 2/33/20.

Un tel fil présente une masse linéique au repos de 480 dtex, avec donc une teneur en élasthanne de 16 %.

La caractéristique d'allongement de ce fil est donné en II sur la figure 2, qui rend compte de la différence importante de nervosité par rapport à un fil de bas de contention classe I (caractéristique I de la figure 2).

On a tricoté avec ce fil un bas de largeur 9,4/13,2/16,3 mm.

Après formage et teinture, ce bas procure une pression à la cheville de 4,9 mmHg et à la cuisse de 2,7 mmHg, soit une dégressivité de 55 %.

Cette valeur de dégressivité n'étant pas encore satisfaisante, la largeur a été modifiée, avec des valeurs de 10,5/14/17 mm. Après formage et teinture, la pression à la cheville mesurée est de 3,7 mmHg pour une pression à la cuisse de 2,4 mmHg, soit une dégressivité de 65 %.

Un autre essai a été mené avec le même choix de fil et les mêmes valeurs de largeur que ci-dessus, mais avec formage et sans teinture : la pression à la cheville y est alors de 2,8 mmHg et la dégressivité de 118 %.

Un autre essai encore a été mené, en modifiant les largeur au mollet à la cuisse, avec des dimensions de largeur 10,5/12,6/15,9 mm. Après formage et avant teinture la pression à la cheville est de 3,9 mmHg et la dégressivité de 74 %. Après teinture, la pression à la cheville est de 3,0 mmHg et la dégressivité de 103 %.

L'ajout final d'un adoucissant après teinture modifie légèrement ces paramètres : avec un adoucissement à 6 % (au lieu de 1,5 % dans un processus normal), la pression à la cheville est réduite à 2,8 mmHg et la dégressivité à 87 %.

Ces divers essais montrent qu'il est possible, une fois le fil de trame choisi sur les critères indiqués plus haut, d'obtenir un ajustement fin des valeurs de pression à la cheville et de dégressivité en jouant sur plusieurs paramètres tels que largeur de tricotage cheville/mollet/cuisse, température de formage, paramètres de la teinture, addition d'adoucissant et teneur de celui-ci, etc.

En tout état de cause, pour conserver une masse linéique proche de celle d'un fil de bas de contention et avec une capacité d'élongation comparable, il apparaît nécessaire de jouer sur la nervosité du fil, choisie notablement plus faible afin que le bas soit inefficace sur le plan hémodynamique.

On peut par ailleurs envisager de diminuer encore le titre de l'âme d'élasthanne en choisissant par exemple une âme de 44 dtex double couverture, chaque fil de couverture étant lui-même constitué de deux fils de 78 dtex de polyamide.

## Revendications

1. Une orthèse de contention telle qu'un bas médical, réalisée par tricotage d'un fil de tricot (10) combiné à un fil de trame (12) extensible, **caractérisée en ce que** le fil de trame (12) présente :
- une masse linéique, mesurée sans tension, comprise entre 250 et 600 dtex, de préférence comprise entre 350 et 500 dtex,
- une proportion de matériau élastique inférieure à 25 %, de préférence inférieure à 20 %, du titre total du fil , et
- une capacité d'élongation comprise entre 200 et 350 %, de préférence comprise entre 200 et 300 %,
ladite orthèse étant une orthèse de type placebo apte à exercer au niveau de la cheville une pression de contention inférieure à 8 mmHg, de préférence inférieure à 5 mmHg.

2. L'orthèse de la revendication 1, où ladite orthèse est apte à exercer une contention dont le rapport des pressions entre cuisse et cheville est inférieur à 125 %, de préférence inférieur à 110 %.

3. L'orthèse de la revendication 1, où ladite orthèse est apte à exercer une contention dont le rapport des pressions entre cuisse et cheville est au moins égal à 80 %.

## Patentansprüche

1. Stützorthese, wie ein medizinischer Strumpf, verwirklicht durch Stricken eines Strickfadens (10), kombiniert mit einem dehnbaren Schussfaden (12), **dadurch gekennzeichnet, dass** der Schussfaden (12) aufweist:
- eine längenbezogene Masse, gemessen ohne Spannung, die zwischen 250 und 600 dtex liegt, vorzugsweise zwischen 350 und 500 dtex,
- einen Anteil von elastischem Material von weniger als 25%, vorzugsweise von weniger als 20%, der Gesamttitrierung des Fadens, und
- eine Dehnfähigkeit zwischen 200 und 350%, vorzugsweise zwischen 200 und 300%,
wobei die Orthese eine Orthese vom Placebotyp ist, dazu geeignet, auf Höhe des Fußknöchels einen Kompressionsdruck von weniger als 8 mmHg, vorzugsweise geringer als 5 mmHg, auszuüben.

2. Orthese nach Anspruch 1, wo die Orthese in der Lage ist, eine Kompression auszuüben, von der das Verhältnis der Drücke zwischen Oberschenkel und Fußknöchel geringer als 125% ist, vorzugsweise geringer als 110%.

3. Orthese nach Anspruch 1, wo die Orthese in der Lage ist, eine Kompression auszuüben, von der das Verhältnis der Drücke zwischen Oberschenkel und Fußknöchel wenigstens gleich 80% ist.

## Claims

1. A compressive orthosis such as a surgical stocking made by knitting a knitting yarn (10) together with an extensible weft yam (12), **characterized in that** the weft yam (12) presents:
- a mass per unit length, measured under no tension, lying in the range 250 dtex to 600 dtex, and preferably lying in the range 350 dtex to 500 dtex,
- a proportion of elastic material below 25%, and preferably below 20% of the total weight of the yarn, and
- an elongation capacity lying in the range 200% to 350%, and preferably lying in the range 200% to 300%,
said orthosis being a placebo type orthosis suitable for exerting compressive pressure at the ankle of less than 8 mmHg, and preferably less than 5 mmHg.

2. The orthosis of claim 1, wherein said orthosis is adapted to exert a compression which ratio of pressures between the thigh and the ankle is less than 125%, and preferably less than 110%.

3. The orthosis of claim 1, wherein said orthosis is adapted to exert a compression which ratio of pressures between the thigh and the ankle is not less than 80%.
